# EUROPEAN PATENT APPLICATION

(11) **EP 4 552 702 A1**
(43) Date of publication of application: **14.05.2025**
(21) Application number: 23835089.6
(22) Date of filing: 17.02.2023
(51) Int. Cl.: A61Q 17/04, A61K 8/06, A61K 8/30, A61K 8/31, A61K 8/37

(54) **COMPOSITION FOR UV ABSORPTION AND METHOD FOR MANUFACTURING SAME**

(30) Priority: 08.07.2022 JP 2022110409
(71) Applicant: FUJI PIGMENT CO., LTD., Kawanishi-shi, Hyogo-ken 666-0015 (JP); GS Alliance Co., Ltd., Kawanishi-shi, Hyogo, 666-0015 (JP)
(72) Inventor: TAKETOMI,Sho, Kawanishi-shi, Hyogo 666-0015 (JP); MORI,Ryohei, Kawanishi-shi, Hyogo 666-0015 (JP)
(74) Representative: HGF
(86) International application number: PCT/JP2023/005774
(87) International publication number: WO 2024/009548

(57) **Abstract**

Provided are a composition for UV absorption that has a superior UV absorption function, and is superior in dispersibility while absorbing, in particular, UVA (wavelength: 315 to 380 nm) and UVB (wavelength: 280 to 315 nm), a method for manufacturing the same, and a UV absorption method using the same. The composition for UV absorption includes a carbon-based quantum dot, an aqueous solvent, a surfactant, and an oily base. Alternatively, the composition for UV absorption includes a carbon-based quantum dot, an aqueous solvent, and a hydrophilic base. The method for manufacturing a composition for UV absorption includes a step of mixing an aqueous dispersion containing a carbon-based quantum dot and an aqueous solvent, a surfactant, and an oily base. The method for manufacturing a composition for UV absorption includes a step of mixing an aqueous dispersion containing a carbon-based quantum dot and an aqueous solvent, and a hydrophilic base.

## Description

### TECHNICAL FIELD

The present invention relates to a composition for UV absorption that can be used for products such as cosmetics, a method for manufacturing the same, and a UV absorption method using the same.

### BACKGROUND ART

UV rays, namely, electromagnetic waves (light) in a region of about 10 to 400 nm cause not only sunburn but also "photoaging" (a phenomenon in which normal regeneration of the skin cannot proceed in time due to DNA damage or destruction of connective tissue caused by UV rays, and aging proceeds) on our skin. The sunlight includes UV rays called UVA (wavelength: 315 to 380 nm), UVB (wavelength: 280 to 315 nm), and UVC (wavelength: 200 to 280 nm). The UVC having a short wavelength hardly reaches the ground surface, but the UVA and the UVB reach the ground surface. As a result, an aged appearance over age appears on the skin, and a future risk of skin cancer is also included.

Various sunscreen materials have been developed so far. Typical examples thereof include inorganic UV reflectors such as titanium oxide and zinc oxide. Titanium oxide particularly reflects UV rays having a wavelength of 260 to 400 nm mainly including UVB (wavelength: 280 to 315 nm). Zinc oxide reflects the same wavelength as the above, but is superior in reflection of UVA (wavelength: 315 to 380 nm) to titanium oxide. Therefore, the two have been mixed and used as an inorganic UV reflector. For example, Patent Document 1 discloses a UV absorber in which the surface of a flaky substrate is coated with ultrafine zinc oxide particles having an average particle size of 100 nm or less and which has high transparency and superior dispersibility.

On the other hand, sunscreens that have increased since 2006 contain organic UV absorbers. Currently, about half of the market has been occupied by organic UV absorbers. For example, Patent Document 2 discloses a sunscreen aerosol cosmetic containing ethylhexyl methoxycinnamate and the like as an organic UV absorber.

### PRIOR ART DOCUMENT

### PATENT DOCUMENTS

Patent Document 1: JP-A-11-302625
Patent Document 2: JP-A-2021-080203

### SUMMARY OF THE INVENTION

### PROBLEMS TO BE SOLVED BY THE INVENTION

However, for example, in the inorganic UV absorber containing ultrafine zinc oxide particles in Patent Document 1, a powder is likely to aggregate over a long period of time, and therefore there is room for further improvement from the viewpoint of dispersibility and the like.

Further, as a case where an inorganic UV reflector is not used, for example, there is the sunscreen containing an organic UV absorber in Patent Document 2. There are many types of organic UV absorbers, but they are different in wavelength range of rays which the organic UV absorbers can absorb, and therefore a wide range cannot be covered by a single component. Therefore, it is necessary to mix at least about three types thereof and the work is complicated, so that there is room for further improvement.

Therefore, an object of the present invention is to provide a composition for UV absorption that has a superior UV absorption function, and is superior in dispersibility while absorbing, in particular, UVA (wavelength: 315 to 380 nm) and UVB (wavelength: 280 to 315 nm), a method for manufacturing the same, and a UV absorption method using the same.

### MEANS FOR SOLVING THE PROBLEMS

The present inventors have extensively conducted studies for achieving the above object, and resultantly have found that when a carbon-based quantum dot is well dispersed in an oily base or a hydrophilic base, a superior UV absorption function is exhibited, leading to accomplishment of the present invention.

That is, the present invention includes the following aspects.

1. A composition for UV absorption comprising a carbon-based quantum dot, an aqueous solvent, a surfactant, and an oily base.
2. A composition for UV absorption comprising a carbon-based quantum dot, an aqueous solvent, and a hydrophilic base.
3. The composition for UV absorption according to 1. or 2., wherein a content of the carbon-based quantum dot is 0.001 to 10 w/w% based on an overall amount of the composition.
4. The composition for UV absorption according to 1. or 3., wherein the surfactant is one or more members selected from the group consisting of nonionic surfactant, anionic surfactant, cationic surfactant, and amphoteric surfactant.
5. The composition for UV absorption according to 1., 3. or 4., wherein the oily base is one or more members selected from the group consisting of hydrocarbons, fatty acids, higher alcohol, ester oil, fat and oil, wax, siloxane, and silicone.
6. The composition for UV absorption according to any one of 2. to 4., wherein the hydrophilic base is one or more members selected from the group consisting of saccharides, water-soluble polymer, polyhydric alcohol, cellulose derivative, glycol ether, and lower alcohol.
7. A composition for UV absorption, wherein a form of a preparation into which the composition for UV absorption described in any one of 1. to 6. is processed is one or more selected from the group consisting of solution, suspension, emulsion, cream, ointment, gel, liniment, spray, aerosol, cataplasm, sheet, powder, and lotion.
8. A cosmetic comprising the composition for UV absorption according to any one of 1. to 7.
9. A method for manufacturing a composition for UV absorption, comprising a step of mixing an aqueous dispersion containing a carbon-based quantum dot and an aqueous solvent, a surfactant, and an oily base.
10. A method for manufacturing a composition for UV absorption, comprising a step of mixing an aqueous dispersion containing carbon-based quantum dot and an aqueous solvent, and a hydrophilic base.
11. A UV absorption method using a composition for UV absorption containing a carbon-based quantum dot, an aqueous solvent, a surfactant, and an oily base.
12. A UV absorption method using a composition for UV absorption containing a carbon-based quantum dot, an aqueous solvent, and a hydrophilic base.

### EFFECT OF THE INVENTION

According to the composition for UV absorption of the present invention, it is possible to provide a composition for UV absorption being superior in dispersibility while having a superior UV absorption function. In addition, according to the method for manufacturing a composition for UV absorption of the present invention, it is possible to obtain a composition for UV absorption being superior in dispersibility while having a superior UV absorption function. Further, according to the UV absorption method of the present invention, it is possible to provide a UV absorption method using a composition for UV absorption being superior in dispersibility while having a superior UV absorption function.

### BRIEF DESCRIPTION OF THE DRAWINGS

Fig. 1 is a photograph showing dispersibility immediately after filling of the composition for UV absorption of Example 1 obtained using a graphene quantum dot.
Fig. 2 is a photograph showing dispersibility one month after filling of the composition for UV absorption of Example 1 obtained using a graphene quantum dot.
Fig. 3 is a photograph showing dispersibility immediately after filling of the composition for UV absorption of Comparative Example 1 containing no surfactant.
Fig. 4 is a graph showing the absorption distribution due to a QD concentration of an aqueous dispersion of graphene quantum dots (in a state before being dispersed in petrolatum).
Fig. 5 is a graph showing the absorption distribution due to a QD concentration of an aqueous dispersion of graphene quantum dots (in a state after being dispersed in petrolatum) with respect to petrolatum (control).
Fig. 6 is a graph showing the absorption distribution due to a QD concentration of an aqueous dispersion of graphene quantum dots (in a state after being dispersed in petrolatum) with respect to petrolatum (control) after a lapse of two months.

### MODE FOR CARRYING OUT THE INVENTION

### <Composition for UV absorption>

A composition for UV absorption of the present invention contains a carbon-based quantum dot, an aqueous solvent, a surfactant, and an oily base. Another composition for UV absorption of the present invention contains a carbon-based quantum dot, an aqueous solvent, and a hydrophilic base. The compositions for UV absorption of the present invention can be used for products such as cosmetics. In the following, modes for carrying out the present invention will be described in detail. The present invention, however, is not limited to the following embodiments.

### (Carbon-based quantum dot)

A composition for UV absorption of the present invention contains a carbon-based quantum dot. A quantum dot refers to any nanoscale particle having unique optical characteristics following quantum chemistry and quantum mechanics, and has characteristic emission characteristics depending on the particle size because the optical characteristics can be adjusted by the particle size. In the present invention, among quantum dots, a carbon-based quantum dot having light emission characteristics depending on the particle size due to a π bond between carbon atoms can be used.

Examples of the carbon-based quantum dot include graphene quantum dots, which have a graphene structure, carbon quantum dots, which do not have a graphene structure, and quantum dots obtained by chemically modifying these. From the viewpoint of practical use, one or more members selected from the group consisting of graphene quantum dots and carbon quantum dots are preferable.

These carbon-based quantum dots are commercially available from Sigma-Aldrich, Fuji Pigment Co., Ltd., GS Alliance Co., Ltd., Funakoshi Co., Ltd., Kishida Chemical Co., Ltd., and so on, and any of them can be used. From the viewpoint of practicality, it is preferable to use those in the form of an aqueous dispersion in which a carbon-based quantum dot is dispersed in an aqueous solvent described later, and those in such a form can also be obtained as commercial products.

A content of the carbon-based quantum dot based on an overall amount of the composition is not particularly limited at the time of use as a raw material, but is preferably 0.001 w/w% or more and 10 w/w% or less, more preferably 0.01 w/w% or more and 8 w/w% or less, still more preferably 0.05 w/w% or more and 6 w/w% or less, particularly preferably 0.1 w/w% or more and 5 w/w% or less, and especially preferably 0.1 w/w% or more and 3.5 w/w% or less based on the overall amount of the composition from the viewpoint of obtaining appropriate absorbance and practical use. In the present description, the unit of content "w/w%" is synonymous with w/w% meaning "g/100 g". Incidentally, the content of the carbon-based quantum dot at the time of processing into a preparation is preferably the concentration described above, but the concentration at the time of being a raw material may be either equal to the concentration at the time of formulation or higher than that.

### (Graphene quantum dot)

A graphene quantum dot includes a non-functionalized graphene quantum dot, a functionalized graphene quantum dot, a pristine graphene quantum dot, and a combination thereof.

The functionalized graphene quantum dot may have been functionalized with one or more functional groups. The functional groups include an oxygen group, a carboxyl group, a carbonyl group, amorphous carbon, a hydroxy group, an alkyl group, an aryl group, an ester, an amine, an amide, a polymer, poly(propylene oxide), and a combination thereof.

The graphene quantum dot also includes a functionalized graphene quantum dot that is functionalized with one or more alkyl groups. The alkyl groups include a methyl group, an ethyl group, a propyl group, a butyl group, a pentyl group, a hexyl group, a heptyl group, an octyl group, a nonyl group, a decyl group, an undecyl group, and a combination thereof. In some embodiments, the alkyl groups include an octyl group (for example, octylamine).

The graphene quantum dot may be functionalized with one or more types of polymer precursors. For example, the graphene quantum dot may be functionalized with one or more types of monomers (for example, vinyl monomers).

The graphene quantum dot can form a polymer-functionalized graphene quantum dot via functionalization with a polymer precursor to be polymerized. For example, via functionalization of ends of a graphene quantum dot with a polymer precursor to be polymerized, an end-functionalized polyvinyl adduct can be formed.

The graphene quantum dot includes a functionalized graphene quantum dot functionalized with one or more types of hydrophilic functional groups. The hydrophilic functional groups include a carboxyl group, a carbonyl group, a hydroxy group, hydroxyalkyl groups, poly(ethylene glycol), poly(vinyl alcohol), poly(acrylic acid), and a combination thereof.

The graphene quantum dot includes a functionalized graphene quantum dot functionalized with one or more types of hydrophobic functional groups. The hydrophobic functional groups include an alkyl group, an aryl group, and a combination thereof. The hydrophobic functional groups include one or more types of alkylamides or arylamides.

The graphene quantum dot includes an end-functionalized graphene quantum dot. The end-functionalized graphene quantum dot contains the one or more types of hydrophobic functional groups described above. The end-functionalized graphene quantum dot contains one or more types of hydrophobic functional groups such as those described above. The end-functionalized graphene quantum dot also contains one or more types of hydrophilic functional groups such as those described above. The end-functionalized graphene quantum dot includes one or more types of oxygen adducts located on their ends. The end-functionalized graphene quantum dot includes one or more types of amorphous carbon adducts located on their edges.

The graphene quantum dot is functionalized at ends thereof with one or more types of alkyl groups or aryl groups of alkylamides or arylamides. The end functionalization of the graphene quantum dot using alkyl groups or aryl groups is performed via a reaction of a carboxylic acid with an alkylamide or an arylamide at the ends of the graphene quantum dot.

The graphene quantum dot includes a pristine graphene quantum dot. The pristine graphene quantum dot includes a graphene quantum dot remaining untreated after synthesis. The pristine graphene quantum dot includes a graphene quantum dot that has not subjected to any additional surface modification after synthesis.

The graphene quantum dot can be obtained from a variety of sources. For example, the graphene quantum dot includes a graphene quantum dot derived from coal, a graphene quantum dot derived from coke, and a combination thereof. The graphene quantum dot includes a graphene quantum dot derived from coke. The graphene quantum dot includes a graphene quantum dot derived from coal. Examples of coal include, but are not limited to, anthracite, bituminous coal, sub-bituminous coal, modified bituminous coal, asphaltene, asphalt, peat, lignite, boiler coal, petrified oil, carbon black, activated carbon, and a combination thereof. The carbon source is bituminous coal. Carbon includes bituminous coal.

The graphene quantum dot can have various diameters. For example, the graphene quantum dot preferably has a diameter in the range of about 1 nm to about 100 nm, more preferably have a diameter in the range of about 1 nm to about 50 nm, and still more preferably have a diameter in the range of about 1 nm to about 20 nm.

The graphene quantum dot can also have various structures. For example, the graphene quantum dot may have a crystalline structure, for example, a crystalline hexagonal structure. The graphene quantum dot may have a single layer or multiple layers, for example, the graphene quantum dot has approximately two layers to approximately four layers.

The graphene quantum dot can also have various quantum yields. The graphene quantum dot preferably has a quantum yield in the range of from about 30 to 80%. In addition, regarding the fluorescence characteristic of the graphene quantum dot in an aqueous dispersion, the emission wavelength is preferably 380 nm to 650 nm for at least any wavelength of 300 nm to 420 nm of excitation light.

The graphene quantum dot may be in the form of powder or in the form of pellets. The graphene quantum dot may be in a liquid state, a dispersion, a solution, or a molten state. From the viewpoint of dispersibility, the graphene quantum dot is preferably in the form of an aqueous dispersion in which the graphene quantum dot is dispersed in an aqueous solvent described later, and a graphene quantum dot in such a form can be obtained also as a commercially available product.

Various methods can be utilized to form the graphene quantum dot. For example, the process of forming a graphene quantum dot may involve exposing a carbon source to an oxidizing agent, resulting in forming a graphene quantum dot. The carbon source includes coal, coke, and a combination thereof.

The oxidizing agent includes an acid, and the acid includes sulfuric acid, nitric acid, phosphoric acid, hypophosphorous acid, fuming sulfuric acid, hydrochloric acid, oleum, chlorosulfonic acid, and a combination thereof. The oxidizing agent also includes potassium permanganate, sodium permanganate, hypophosphorous acid, nitric acid, sulfuric acid, hydrogen peroxide, and a combination thereof. A preferred oxidizing agent is a mixture of potassium permanganate, sulfuric acid and hypophosphorous acid.

By sonicating the carbon source in the presence of an oxidizing agent, the carbon source is exposed to the oxidizing agent. Heating the carbon source in the presence of an oxidizing agent is included. The heating is performed at a temperature of at least about 100°C.

The use of another method of forming a graphene quantum dot can also be envisaged. For example, a further method of forming a graphene quantum dot is disclosed in the international patent application PCT/US2014/036604. Further suitable methods for manufacturing a graphene quantum dot are also disclosed in the following references: ACS Appl. Mater. Interfaces 2015, 7, 7041-7048; and Nature Commun. 2013, 4: 2943, 1-6.

### (Carbon quantum dot)

A carbon quantum dot is a quantum dot having no cyclic structure like graphene. The carbon quantum dot is more easily affected by the pH value than the graphene quantum dot, and has a property that the emission intensity and the peak position vary.

The carbon quantum dot can have various diameters. For example, the carbon quantum dot preferably has a diameter in the range of about 1 nm to about 100 nm, more preferably have a diameter in the range of about 1 nm to about 50 nm, and still more preferably have a diameter in the range of about 1 nm to about 30 nm.

The carbon quantum dot can also have various quantum yields. The carbon quantum dot preferably has a quantum yield in the range of from about 20 to 50%. In addition, regarding the fluorescence characteristic of the carbon quantum dot in an aqueous dispersion, the emission wavelength is preferably 380 nm to 600 nm for at least any wavelength of 300 nm to 420 nm of excitation light.

The method for manufacturing the carbon quantum dot is not much different from the method for manufacturing the graphene quantum dot, and only the difference is whether or not the raw materials to be used and the manufacturing conditions are easy to form a graphene structure.

Therefore, the carbon-based quantum dot including both types can be manufactured, for example, by a method in which a carbon target is laser-ablated and then subjected to a chemical treatment (JP-A-2012-501863), a method in which carbon-based quantum dots are manufactured from soot of candles (H. Liu, et al., Angew. Chem. Int. Ed. 2007, 46, 6473-6475.), a method in which carbon-based quantum dots are manufactured by chemically treating a graphite oxide (G. Eda, et al., Adv. Mater. 2010, 22, 505-509.), a method in which carbon-based quantum dots are manufactured via a chemical reaction using a graphite oxide as a precursor (JP-A-2012-136566), a method in which carbon-based quantum dots are manufactured via a conversion reaction of fullerene (J. Lu, et al., Nature Nanotech. 2011, 6, 247-252.), or a method in which carbon-based quantum dots are manufactured by chemically treating a cheaper carbon raw material such as carbon fiber or activated carbon (J. Peng, et al., Nano Lett. 2012, 12, 844-849, Z. A. Qiao, ChemCommun. 2010, 46, 8812-8814, Y. Dong, et al., Chem. Mater. 2010, 22, 5895-5899.).

These techniques are roughly classified as top-down techniques, but carbon-based quantum dots can also be manufactured by bottom-up techniques in which carbon quantum dots are manufactured via polymerization of organic precursor molecules (G. A. Ozin, et al., J. Mater. Chem., 2012, 22, 1265-1269.).

Alternatively, carbon-based quantum dots can also be manufactured by a method for manufacturing carbon quantum dots, including the steps of: mixing a carbon material with hydrogen peroxide to make carbon to undergo a decomposition reaction by hydrogen peroxide, thereby preparing a carbon quantum dot generation liquid; and separating carbon quantum dots and hydrogen peroxide in the carbon quantum dot generation liquid from each other to stop the decomposition reaction and obtain carbon quantum dots (JP-A-2014-133685).

The carbon quantum dot may be in the form of powder or in the form of pellets. The carbon quantum dot may be in a liquid state, a dispersion, a solution, or a molten state. From the viewpoint of dispersibility, the carbon quantum dot is preferably in the form of an aqueous dispersion in which the carbon quantum dot is dispersed in an aqueous solvent described later, and a carbon quantum dot in such a form can be obtained also as a commercially available product.

### (Aqueous solvent)

The composition for UV absorption of the present invention contains an aqueous solvent. It is preferable for the aqueous solvent in the present invention to be water or to mainly contain water in consideration of the influence on the environment and the human body. The content of water based on the overall amount of the aqueous solvent is preferably 80 w/w% or more and 100 w/w% or less, more preferably 85 w/w% or more and 99.9 w/w% or less, and still more preferably 90 w/w% or more and 99.5 w/w% or less based on the overall amount of the aqueous solvent. The water to be used in the present invention is not particularly limited, and examples thereof include tap water, purified water, distilled water, ion-exchanged water, pure water, and ultrapure water.

In addition, the aqueous solvent in the present invention may contain conventional well-known optional components without particular limitation, and may contain, for example, an optional substance such as a dispersant, an acid, a salt, or an organic solvent. Examples of the organic solvent preferably include organic solvents that are miscible with water, and examples thereof include methanol, ethanol, isopropanol, acetone, acetonitrile, propionitrile, tetrahydrofuran, 1,4-dioxane, methyl isobutyl ketone, methyl ethyl ketone, γ-butyl lactone, propylene carbonate, sulfolane, nitromethane, N,N-dimethylformamide, N-methylacetamide, dimethyl sulfoxide, dimethyl sulfone, N-methylpyrrolidone, benzene, toluene, xylene, methylene chloride, chloroform, and dichloroethane.

The content of the aqueous solvent based on the overall amount of the composition is not particularly limited, but even in the case of using an oily base in the composition or even in the case of using a hydrophilic base in the composition, the content of the aqueous solvent is preferably 0.01 w/w% or more and 50 w/w% or less, more preferably 0.05 w/w% or more and 40 w/w% or less, and still more preferably 0.1 w/w% or more and 30 w/w% or less based on the overall amount of the composition.

### (Aqueous dispersion)

In the present invention, from the viewpoint of stability, a form of an aqueous dispersion containing the above-described carbon-based quantum dot and the above-described aqueous solvent is preferable, and as described above, one commercially available as an aqueous dispersion can be used as it is. In addition, to improve dispersibility, the aqueous dispersion may be further diluted with the above-described aqueous solvent to adjust the concentration of the carbon-based quantum dot before use. The content of the carbon-based quantum dot based on the overall amount of the aqueous dispersion is preferably 1 w/w% or more and 30 w/w% or less, and more preferably 5 w/w% or more and 20 w/w% or less based on the overall amount of the aqueous dispersion from the viewpoint of production efficiency and workability. The aqueous dispersion may contain conventional well-known optional components without particular limitation, and may contain an optional substance such as a dispersant, an acid, a salt, or an organic solvent. The aqueous dispersion in the present invention can be manufactured, for example, by mixing and stirring the carbon-based quantum dot, an aqueous solvent, and other optional components, as necessary, by a method commonly used in the art.

### (Surfactant)

The composition for UV absorption of the present invention contains a surfactant when an oily base is used in the composition. On the other hand, when a hydrophilic base is used in a composition, the composition also may contain a surfactant. The surfactant in the present invention is not particularly limited, and those well-known in the art can be used, but from the viewpoint of improving dispersibility, the surfactant is preferably one or more members selected from the group consisting of a nonionic surfactant, an anionic surfactant, a cationic surfactant, and an amphoteric surfactant.

The nonionic surfactant is not particularly limited, and those well-known in the art can be used, and examples thereof include polyoxyalkylene alkyl ethers, polyoxyalkylene alkenyl ethers, alkyl glyceryl ethers, alkenyl glyceryl ethers, fatty acid sucrose esters, glycerin fatty acid esters, polyglycerin fatty acid esters, polyethylene glycol fatty acid esters, propylene glycol fatty acids, ester higher fatty acids, sorbitan fatty acid esters, polyoxyethylene sorbitan fatty acid esters, polyoxyethylene sorbitol fatty acid esters, polyoxyethylene fatty acid esters, mono- or diethanolamides, polyoxyethylene hydrogenated castor oils, alkylsaccharides, alkylamine oxides, alkylamidoamine oxides, polyoxyalkylene-modified silicones, polyoxyalkylene-modified organopolysiloxanes, and polyoxyalkylene-alkyl co-modified organopolysiloxanes. From the viewpoint of improving dispersibility, it is preferable to use an ether-type nonionic surfactant such as a polyoxyalkylene alkyl ether, a polyoxyalkylene alkenyl ether, an alkyl glyceryl ether, or an alkenyl glyceryl ether, or an ester-type nonionic surfactant such as a fatty acid sucrose ester, a glycerin fatty acid ester, a polyglycerin fatty acid ester, a polyethylene glycol fatty acid ester, a propylene glycol fatty acid, an ester higher fatty acid, a sorbitan fatty acid ester, a polyoxyethylene sorbitan fatty acid ester, a polyoxyethylene sorbitol fatty acid ester, or a polyoxyethylene fatty acid ester. These may be used singly or two or more of them may be used in combination.

Examples of the sorbitan fatty acid ester include sorbitan monopalmitate, sorbitan monostearate, sorbitan monooleate, sorbitan sesquioleate, sorbitan coconut-oil fatty acid, and sorbitan tristearate. Examples of commercially available products thereof include RHEODOL SP-P10 as sorbitan monopalmitate, RHEODOL SP-S10 V as sorbitan monostearate, RHEODOL SP-O10V and RHEODOL AO-10V as sorbitan monooleate, RHEODOL AO-15V as sorbitan sesquioleate, RHEODOL SP-L10 as sorbitan coconut-oil fatty acid, and SP-S30 V as sorbitan tristearate (all manufactured by Kao Corporation).

The anionic surfactant is not particularly limited, and those well-known in the art can be used, and examples thereof include sulfuric acid-based surfactants, sulfonic acid-based surfactants, carboxylic acid-based surfactants, and phosphoric acid-based surfactants. Examples of the sulfuric acid-based surfactants include alkyl sulfates, polyoxyalkylene alkyl ether sulfates, polyoxyalkylene alkenyl ether sulfates, and sulfosuccinic acid alkylene alkyl phenyl ether sulfates. Examples of the sulfonic acid-based surfactants include alkanesulfonic acid salts. Examples of the carboxylic acid-based surfactants include alkyl ether carboxylic acids or salts thereof, and polyoxyethylene alkyl ether carboxylic acids or salts thereof. Examples of the phosphoric acid-based surfactants include monoalkyl phosphates. These may be used singly or two or more of them may be used in combination.

For example, as a polyoxyethylene alkyl ether carboxylic acid or a salt thereof, specifically, one or two or more selected from among polyoxyethylene lauryl ether carboxylic acid or a salt thereof, polyoxyethylene myristyl ether carboxylic acid or a salt thereof, and polyoxyethylene palmityl ether carboxylic acid or a salt thereof are preferably contained. Examples of commercially available products thereof include KAO AKYPO RLM-45NV, KAO AKYPO RLM-45, KAO AKYPO RLM-100NV, and KAO AKYPO RLM-100 (all manufactured by Kao Corporation).

The cationic surfactant is not particularly limited, and those well-known in the art can be used, and examples thereof include quaternary ammonium salts and alkylamine salts. These may be used singly or two or more of them may be used in combination.

Examples of the quaternary ammonium salts include lauryltrimethylammonium chloride, cetyltrimethylammonium chloride, stearyltrimethylammonium chloride, distearyldimethylammonium chloride, alkyl(having 11 or more and 23 or less carbon atoms)benzyldimethylammonium chloride, alkyltrimethylammonium salts, dialkyldimethylammonium salts, and alkylbenzyldimethylammonium salts. Examples of these commercially available products include QUARTAMIN 24P, QUARTAMIN 60W, QUARTAMIN 86P CONC, QUARTAMIN 86W, QUARTAMIN D86P, SANISOL C, and SANISOL B-50 (all manufactured by Kao Corporation).

Examples of the alkylamine salts include alkylamine salts having an alkyl group having 11 or more and 23 or less carbon atoms (for example, the salts are acetates). Specific examples of the alkylamine salts include coconut amine acetate and stearyl amine acetate. Examples of these commercially available products include ACETAMIN 24 and ACETAMIN 86 (both manufactured by Kao Corporation).

The amphoteric surfactant is not particularly limited, and those well-known in the art can be used, and examples thereof include betaine acetate-type surfactants such as betaine lauryldimethylaminoacetate, amine oxide-type surfactants such as lauryldimethylamine oxide, imidazolinium betaine-type surfactants such as 2-alkyl-N-carboxymethyl-N-hydroxyethyl imidazolinium betaine, amidobetaine-type surfactants such as amidopropyl betaine laurate, and sulfobetaine-type surfactants such as lauryl hydroxysulfobetaine. These may be used singly or two or more of them may be used in combination.

As the surfactant in the present invention, surfactants like those described above may be used singly or two or more of them may be used in combination. When an oily base is used in the composition, the content of the surfactant based on the overall amount of the composition (when two or more surfactants are used in combination, the total content thereof) is preferably 0.01 w/w% or more and 5.0 w/w% or less, more preferably 0.1 w/w% or more and 4.5 w/w% or less, and still more preferably 0.5 w/w% or more and 4.0 w/w% or less based on the overall amount of the composition from the viewpoint of improving dispersibility. Even when a hydrophilic base is used in the composition, a surfactant may be added, and in this case, the content of the surfactant is not particularly limited, but may be adjusted to the amount described above.

In the composition for UV absorption of the present invention, the blending amount ratio of the surfactant to the carbon-based quantum dot is preferably 0.01 parts by mass or more and 20.0 parts by mass or less, and more preferably 0.1 parts by mass or more and 10.0 parts by mass or less, to 1 part by mass of the overall content of the carbon-based quantum dot.

### (Oily base)

The composition for UV absorption of the present invention preferably contains an oily base. In the present description, the term "oily" means that the solubility in water is 1 w/w% or less. The oily base in the present invention is not particularly limited, and those well-known in the art can be used, and examples thereof include hydrocarbons, fatty acids, higher alcohol, ester oil, fat and oil, wax, siloxane, and silicone. In the present invention, the oily base is preferably one or more members selected from the group consisting of hydrocarbon, fatty acids, higher alcohol, ester oil, fat and oil, wax, siloxane, and silicone.

Examples of the hydrocarbon include paraffin, isoparaffin, liquid paraffin, liquid isoparaffin, squalane, petrolatum, α-olefin oligomer, light liquid paraffin, and light liquid isoparaffin. These may be used singly or two or more of them may be used in combination. Petrolatum is generally obtained by decoloring and purifying a mixture of hydrocarbons obtained from petroleum, and most of petrolatum contains paraffin having a branched chain (isoparaffin, etc.) and alicyclic hydrocarbon (cycloparaffin, naphthene, etc.).

Examples of the fatty acids include lauric acid, myristic acid, palmitic acid, stearic acid, behenic acid, isostearic acid, and oleic acid. These may be used singly or two or more of them may be used in combination.

In the present description, the term "higher alcohol" means an alcohol having 10 or more and 30 or less carbon atoms. Examples of the higher alcohol include cetanol, stearyl alcohol, cetostearyl alcohol, lauryl alcohol, oleyl alcohol, isostearyl alcohol, behenyl alcohol, cholesterol, and phytosterol. These may be used singly or two or more of them may be used in combination.

Examples of the ester oil include isopropyl myristate, cetyl octoate, octyldodecyl myristate, isopropyl palmitate, isopropyl isostearate, octyl palmitate, diisopropyl adipate, diisopropyl sebacate, isononyl isononanoate, isotridecyl isononanoate, cetyl lactate, isostearyl isostearate, cholesteryl 12-hydroxystearate, cholesteryl oleate, macadamia nut fatty acid phytosteryl ester, phytosteryl oleate, dextrin palmitate, stearoyl inulin, hydrogenated jojoba oil, dipentaerythritol fatty acid ester, neopentyl glycol dicaprate, cyclohexane-1,4-dicarboxylic acid bisethoxy diglycol, tri(2-ethylhexyl) trimellitate, pentaerythritol tetra(2-ethylhexanoate), glycerin tri(2-ethylhexanoate), glyceryl tri(caprylate/caprate), glyceryl tri(caprylate/caprate/myristate/stearate), phytosteryl/octyldodecyl lauroyl glutamate, octyldodecyl/phytosteryl/behenyl lauroyl glutamate, 2-ethylhexyl succinate, triethyl citrate, and phytosteryl/isostearyl/cetyl/stearyl/behenyl dimer dilinoleate. These may be used singly or two or more of them may be used in combination.

Examples of the fat and oil include vegetable fats and oils such as avocado oil, macadamia nut oil, corn oil, olive oil, jojoba oil, grape seed oil, sunflower oil, almond oil, sesame oil, cacao butter, soybean oil, peanut oil, evening primrose oil, rapeseed oil, cottonseed oil, palm oil, palm kernel oil, coconut oil, sunflower oil, and safflower oil, and animal fats and oils such as beef tallow, lard, fish oil, milk fat, horse fat, egg yolk oil, mink oil, and turtle oil. These may be used singly or two or more of them may be used in combination.

Examples of the wax include candelilla wax, carnauba wax, beeswax, spermaceti wax, and orange roughly oil. These may be used singly or two or more of them may be used in combination.

Examples of the siloxane include methylpolysiloxane, methylphenylpolysiloxane, decamethylcyclopentasiloxane, methylcyclopentasiloxane, highly polymerized methylpolysiloxane, octamethylcyclotetrasiloxane, methylhydrogenpolysiloxane, and crosslinked methylpolysiloxane. These may be used singly or two or more of them may be used in combination.

Examples of the silicone include alkyl-modified silicones such as methyl trimethicone, dimethiconol, dimethiconol crosspolymer, and caprylyl methicone, crosslinked alkyl-modified silicones, acrylic silicones, silicone resins, and cyclic silicones. These may be used singly or two or more of them may be used in combination.

As the oily base in the present invention, oily bases like those described above may be used singly or two or more of them may be used in combination. The content of the oily base based on the overall amount of the composition (when two or more oily bases are used in combination, the total content thereof) is preferably 50 w/w% or more and 99 w/w% or less, more preferably 60 w/w% or more and 97 w/w% or less, and still more preferably 70 w/w% or more and 95 w/w% or less based on the overall amount of the composition from the viewpoint of improving dispersibility.

In the composition for UV absorption of the present invention, the blending amount ratio of the oily base to the carbon-based quantum dot is preferably 5 parts by mass or more and 700 parts by mass or less, more preferably 7 parts by mass or more and 650 parts by mass or less, and still more preferably 10 parts by mass or more and 600 parts by mass or less, to 1 part by mass of the overall content of the carbon-based quantum dot.

### (Hydrophilic base)

The composition for UV absorption of the present invention preferably contains a hydrophilic base. In the present description, the term "hydrophilic" means that the solubility in water is more than 50 w/w%. The hydrophilic base in the present invention is not particularly limited, and those well-known in the art can be used, and examples thereof include saccharides, water-soluble polymer, polyhydric alcohol, cellulose derivative, glycol ether, and lower alcohol. In the present invention, the hydrophilic base is preferably one or more members selected from the group consisting of saccharides, water-soluble polymer, polyhydric alcohol, cellulose derivative, glycol ether, and lower alcohol.

Examples of the saccharides include glucose, sucrose, fructose, sorbitol, maltitol, pentaerythritol, xylitol, trehalose, glucosyltrehalose, and carrageenan. These may be used singly or two or more of them may be used in combination.

Examples of the water-soluble polymer include polyvinylpyrrolidone, ethylene glycol triisostearate, polyoxyethylene (20) methyl glucoside triisostearate, bentonite, macrogol, and casein sodium. These may be used singly or two or more of them may be used in combination.

Examples of the polyhydric alcohol include glycerin and polyglycerin (for example, diglycerin and tetraglycerin). Examples of the polyhydric alcohol also include glycols such as ethylene glycol, diethylene glycol, triethylene glycol, polyethylene glycol, propanediol, propylene glycol, dipropylene glycol, polypropylene glycol, isoprene glycol, and butylene glycol. These may be used singly or two or more of them may be used in combination. These may be used singly or two or more of them may be used in combination.

Examples of the cellulose derivative include ethyl cellulose, hydroxypropyl cellulose, and hydroxypropyl methyl cellulose. These may be used singly or two or more of them may be used in combination.

Examples of the glycol ether include ethylene glycol monomethyl ether, ethylene glycol monoethyl ether, ethylene glycol monopropyl ether, diethylene glycol monomethyl ether, diethylene glycol monoethyl ether (ethoxy diglycol), diethylene glycol monopropyl ether, diethylene glycol monobutyl ether, propylene glycol monoethyl ether, propylene glycol monopropyl ether, dipropylene glycol monoethyl ether, and dipropylene glycol monopropyl ether. These may be used singly or two or more of them may be used in combination.

In the present description, the term "lower alcohol" means a monohydric alcohol having 1 to 6 carbon atoms. Examples of the lower alcohols include ethanol, propanol, isopropanol, n-butyl alcohol, s-butyl alcohol, t-butyl alcohol, isobutyl alcohol, pentyl alcohol, and hexyl alcohol. These may be used singly or two or more of them may be used in combination.

As the hydrophilic base in the present invention, hydrophilic bases like those described above may be used singly or two or more of them may be used in combination. The content of the hydrophilic base based on the overall amount of the composition (when two or more hydrophilic bases are used in combination, the total content thereof) is preferably 70 w/w% or more and 99 w/w% or less, more preferably 80 w/w% or more and 97 w/w% or less, and still more preferably 85 w/w% or more and 95 w/w% or less based on the overall amount of the composition from the viewpoint of improving dispersibility.

In the composition for UV absorption of the present invention, the blending amount ratio of the hydrophilic base to the carbon-based quantum dot is preferably 400 parts by mass or more and 700 parts by mass or less, and more preferably 500 parts by mass or more and 600 parts by mass or less, to 1 part by mass of the overall content of the carbon-based quantum dot.

### (Optional additives)

The composition for UV absorption of the present invention may contain, in addition to the components described above, additives that are commonly used in cosmetics, quasi-drugs, and the like as long as the effects of the present invention are not impaired. As such additives, for example, a pH adjuster, a humectant, a thickener, a stabilizer, a chelating agent, a preservative, a fragrance, an antioxidant, a whitening agent, an antiinflammatory agent, a coloring agent, or a use feeling improver may be appropriately blended, as necessary. These additives may be used singly or two or more of them may be used in combination. The content of the additives is not particularly limited as long as a desired UV absorption function can be obtained, but is preferably 0.01 w/w% or more and 20 w/w% or less, and more preferably 0.05 w/w% or more and 15 w/w% or less based on the overall amount of the composition.

### (Form)

The form when the composition for UV absorption of the present invention is processed into a preparation is not particularly limited, and may be arbitrarily chosen according to the application of the composition, and examples thereof include solution, suspension, emulsion, cream, ointment, gel, liniment, spray, aerosol, cataplasm, sheet, powder, and lotion. In the present invention, the form of the composition is preferably one or more members selected from the group consisting of solution, suspension, emulsion, cream, ointment, gel, liniment, spray, aerosol, cataplasm, sheet, powder, and lotion. Such preparations can be prepared by methods conventional in the art.

When the composition for UV absorption of the present invention is processed to form a preparation, the content of the carbon-based quantum dot is preferably adjusted such that a desired UV absorption effect is obtained, and the content of the carbon-based quantum dot is preferably adjusted so as to afford an absorbance of 3.0 or more at a wavelength of 350 nm where the absorbance of petrolatum (control) is 1.

The pH of the composition for UV absorption of the present invention is not particularly limited as long as it is within a physiologically or pharmaceutically acceptable range, but for example, it is preferably 3.5 or more and 8.5 or less, and more preferably 4.0 or more and 7.0 or less.

As the container in which the composition for UV absorption of the present invention is to be filled, a container having a known shape can be used without any limitations. The material of the container is also not particularly limited, and for example, the composition for UV absorption of the present invention can be provided with the composition filled in a container made of such a material as plastic such as polyethylene terephthalate, polyethylene naphthalate, polyarylate, polycarbonate, polyethylene, or polypropylene, or glass.

### (Application)

Since the composition for UV absorption of the present invention is superior in UV absorption function and dispersibility, the composition for UV absorption can be used for various applications in which these properties are required. For example, the composition for UV absorption of the present invention can be used for cosmetics, paints, lubricating oils, or agricultural chemicals. In the present invention, from the viewpoint of enhancing the UV absorption function and improving the dispersibility, it is preferable to prepare a cosmetic containing the composition for UV absorption of the present invention described above. In these applications, the composition for UV absorption of the present invention may be blended, or the components may be individually blended without preparing the composition for UV absorption.

The cosmetic of the present invention can be widely applied as sunscreen cosmetics, makeup cosmetics, skin cleansers, skin care products, hair cosmetics, and the like, but sunscreen cosmetics and makeup cosmetics are preferable from the viewpoint of having a superior UV absorption function. The sunscreen cosmetic may also have a function as a makeup cosmetic like a base cosmetic. More specific examples include base cosmetics; skin cosmetics such as powder foundation, liquid foundation, cream foundation, stick foundation, concealer, loose powder, presto powder, blusher, lipstick, lip gloss, lip liner, lip cream, lip balm, lip stick, eyebrow pencil, eyebrow powder, pencil eye liner, liquid eye liner, mascara, mascara base, eyelash serum, eye shadow, nail color, nail care cosmetic, nail remover, solid soap, face washing foam, face washing powder, cleansing gel, cleansing cream, cleansing oil, point makeup remover, cosmetic water, milky lotion, gel cosmetic liquid, oil, cream, massage cream, hand cream, body cream, pack, peel-off pack, wipe-off/rinse-off pack, gommage, eye care cosmetic, lip care cosmetic, body cleanser, bath agent, body milk, body lotion, hand milk, and hand lotion; and hair cosmetics such as shampoo,, hair treatment, hair styling agent, hair color, and perm solution. The cosmetic of the present invention can be used for protecting the skin and hair from UV rays, but the application amount and use thereof to the skin and the like are not particularly limited, and the cosmetic can be used by applying an appropriate amount thereof to the skin and the like several times a day. As utilization of the present invention in other industrial fields, the present invention can be widely applied to oily paints, oily inks, greases, industrial lubricating oils, OD agents of agricultural chemicals, etc. These can be manufactured in accordance with conventional methods.

### (Method for manufacturing composition for UV absorption)

Hereinafter, the methods for manufacturing the composition for UV absorption of the present invention and the cosmetic of the present invention will be described. The method for manufacturing a composition for UV absorption of the present invention includes a step of mixing an aqueous dispersion containing a carbon-based quantum dot and an aqueous solvent, a surfactant, and an oily base. Alternatively, the method for manufacturing a composition for UV absorption of the present invention includes a step of mixing an aqueous dispersion containing a carbon-based quantum dot and an aqueous solvent, and a hydrophilic base. Note that description of contents similar to those described above may be omitted here.

### (Dilution step)

In the present invention, the aqueous dispersion containing the carbon-based quantum dot and the aqueous solvent is commercially available as described above, and these can be used as received. However, from the viewpoint of improving the dispersibility, the method for manufacturing a composition for UV absorption of the present invention preferably include a dilution step of further diluting the aqueous dispersion with an aqueous solvent or the like such that the content of the carbon-based quantum dot based on the overall amount of the aqueous dispersion is preferably 0.15 w/w% or more and 10 w/w% or less, and more preferably 0.5 w/w% or more and 5 w/w% or less based on the overall amount of the aqueous dispersion.

### (Mixing step)

In the present invention, when an oily base is used in the composition, the method for manufacturing a composition for UV absorption includes a step of mixing an aqueous dispersion containing a carbon-based quantum dot and an aqueous solvent, a surfactant, and an oily base. On the other hand, when a hydrophilic base is used in the composition, the method for manufacturing a composition for UV absorption of the present invention includes a step of mixing an aqueous dispersion containing a carbon-based quantum dot and an aqueous solvent, and a hydrophilic base. Such a mixing step can be performed by a method commonly used in the art. For example, the surfactant described above is added to the aqueous dispersion containing the carbon-based quantum dot, as necessary, followed by sufficient stirring, and then the oily base (or hydrophilic base) described above is added, whereby the respective components can be uniformly mixed. As mixing conditions, it is preferable to perform mixing for about 10 minutes to 3 hours with a stirrer while heating to preferably 40 to 100°C, more preferably 50 to 80°C. The concentration of the carbon-based quantum dot in the final composition can be adjusted by the addition amount of the oily base (or hydrophilic base), and the oily base (or hydrophilic base) or the like can be added such that the content of the carbon-based quantum dot based on the overall amount of the composition is preferably 0.001 w/w% or more and 10 w/w% or less, more preferably 0.01 w/w% or more and 8 w/w% or less, still more preferably 0.05 w/w% or more and 6 w/w% or less, particularly preferably 0.1 w/w% or more and 5 w/w% or less, and especially preferably 0.1 w/w% or more and 3.5 w/w% or less. At the time of mixing, conventional well-known optional components may be included without particular limitations, and for example, an optional substance such as a dispersant, an acid, a salt, or an organic solvent may be included.

### (Method for manufacturing cosmetic)

The cosmetic of the present invention can be manufactured by a method commonly used in the art, for example, by mixing and stirring the composition for UV absorption described above with other components.

### (UV absorption method)

As described above, a UV absorption method of the present invention involves the use of a composition for UV absorption containing a carbon-based quantum dot, an aqueous solvent, a surfactant, and an oily base. As described above, another UV absorption method of the present invention involves the use of a composition for UV absorption containing a carbon-based quantum dot, an aqueous solvent, and a hydrophilic base. Since the details are as described above, the description thereof is omitted here.

### EXAMPLES

Hereinafter, the present invention will be described in detail with reference to Examples, but the present invention is not limited to the following Examples as long as the gist of the present invention is not exceeded. The evaluation items in Examples and the like were measured as follows.

### (1) Measurement of dispersibility

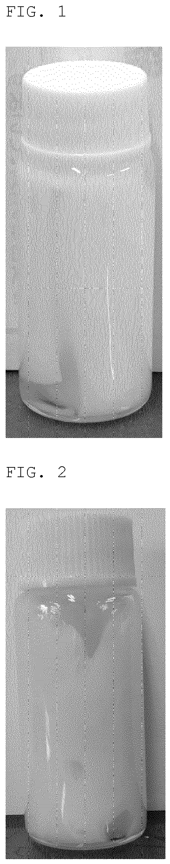

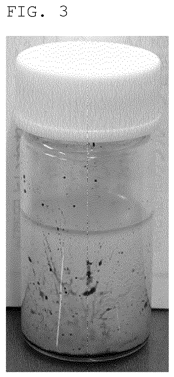

10 mL of each of the compositions for UV absorption obtained in Examples 1 to 8 and Comparative Examples 1 and 2 was weighed and filled in a 20 mL glass bottle, and then the bottle was capped to afford a sample. For each sample, dispersibility immediately after the filling at room temperature was visually confirmed. Further, each sample was stored at rest at room temperature for one month, and dispersibility was visually confirmed. The evaluation results of each sample are shown in Tables 1 and 2 and Figs. 1 to 3. In addition, a photograph immediately after the filling in Example 1 is shown in Fig. 1, a photograph of dispersibility one month after the filling is shown in Fig. 2, and a photograph of dispersibility immediately after the filling in Comparative Example 1 is shown in Fig. 3.
○: Dispersibility is good and the whole of the sample is in a uniform state, and this state remains intact.
△: Dispersibility is good and the whole of the sample is in a uniform state, but slight separation occurs.
×: Dispersibility is poor, and the sample is not in a uniform state as a whole.

### (2) Measurement of absorption spectrum

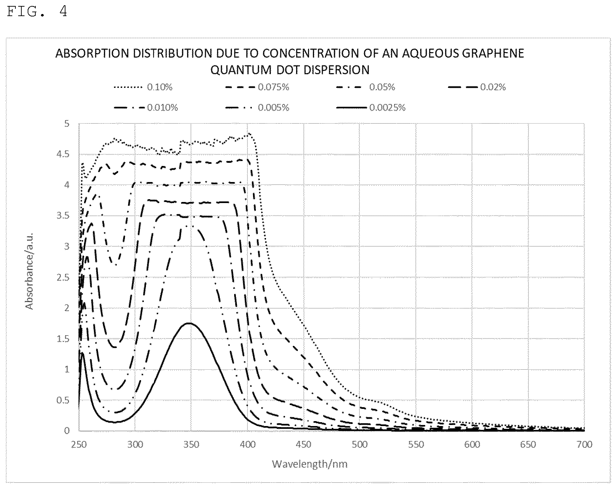

The absorption spectrum of each of the compositions for UV absorption obtained in Examples 1 to 8 and Comparative Examples 1 and 2 was measured using a spectrophotometer (V-770, manufactured by JASCO Corporation). The measurement was performed in a measurement range of 250 nm to 700 nm and 250 nm to 500 nm under measurement conditions of double beams. The measurement results are shown in Figs. 4 to 6. Based on the measurement results, the UV absorption function was evaluated. The UV absorption function was evaluated on the basis of the following criteria. The evaluation results are shown in Tables 1 and 2.
○: The absorbance is 3.0 or more at a wavelength of 350 nm where the absorbance of petrolatum (control) is 1.
△: The absorbance is 2.0 or more and less than 3.0 at a wavelength of 350 nm where the absorbance of petrolatum (control) is 1.
×: The absorbance is less than 2.0 at a wavelength of 350 nm where the absorbance of petrolatum (control) is 1.

Fig. 4 is a graph showing the results measured as follows. The aqueous QD dispersion (QD concentration: 1.5 w/w%) used in Example 1 was further diluted, in a state before being dispersed in petrolatum, with a purified water into the final QD concentrations given in the graph, namely, concentrations of 0.0025 w/w% to 0.10 w/w%, and thereby samples were prepared. The samples were measured using a 1-cm quartz cell with the measuring device and conditions disclosed above.

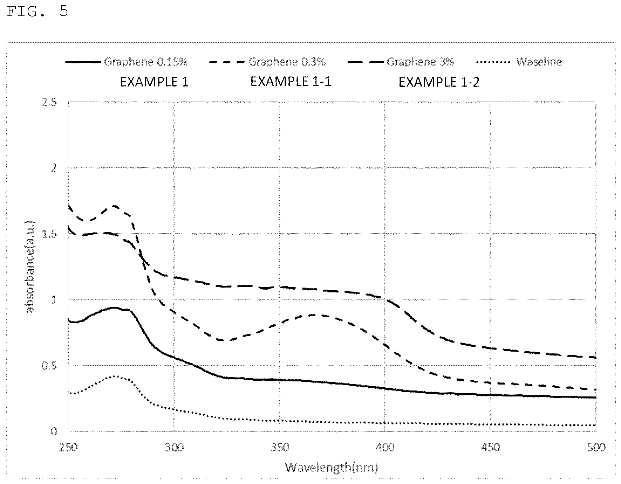

Fig. 5 is a graph showing the result measured as follows. The aqueous QD dispersion (QD concentration: 1.5 w/w%) used in Example 1 was processed to adjust the amount of petrolatum such that the final QD concentration reached the concentrations of 0.15 w/w% to 3 w/w%, which are given in the graph (in the case of 3 w/w%, dilution to the QD concentration of 1.5 w/w% was not performed and a QD concentration of 15 w/w% was used), whereby samples were prepared. Each of the samples was applied to a polyethylene film so as to have a thickness of 25 µm, and then measured with the measuring device and conditions disclosed above. As a control, petrolatum alone was measured in the same manner.

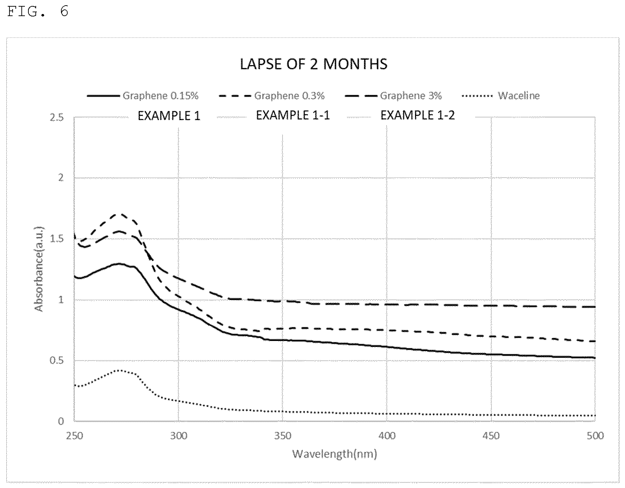

Fig. 6 is similar to Fig. 5, and the absorption spectrum was measured for a sample having lapsed 2 months. The absorption spectrum of the sample having lapsed 2 months is a measurement result of a sample stored without sealing or the like although the sample is in a state in which foreign matters such as dust do not adhere in the state of the preparation thereof. Absorption of 0.5 to 1.0 was confirmed in the range of 300 nm to 450 nm, and deterioration of the UV absorption action was not observed. In particular, at 400 nm or more, the absorption was larger than that at the time of the preparation, but it is considered that this is because moisture was removed and the QD concentration was increased because sealing had not been performed. Although the measured value is not described, it is confirmed that the weight of the sample after a lapse of 2 months has decreased as compared with the sample at the time of preparation.

### <Example 1>

An aqueous dispersion of graphene quantum dots (FUJI QD GRAPHENE 818 manufactured by Fuji Pigment Co., Ltd., quantum dot content (hereinafter, also referred to as QD concentration): 15 w/w%) was diluted 10 times with purified water, and thus an aqueous dispersion was prepared such that the QD concentration was 1.5 w/w% based on the overall amount of the aqueous dispersion. 1 g of a surfactant (RHEODOL AO-10V (nonionic surfactant) manufactured by Kao Corporation) was added to 10 g of the aqueous dispersion (QD concentration: 1.5 w/w%), and the mixture was sufficiently stirred. Thereafter, 89 g of petrolatum (LOSHI BABY WASERIN Pure N manufactured by Cosmetex Roland Co., Ltd.) was added such that the QD concentration was 0.15 w/w% based the overall amount of the composition, and the resulting mixture was sufficiently mixed with a stirrer while heating to 60°C, and thus a composition for UV absorption (QD concentration: 0.15 w/w% based on the overall amount of the composition).

### <Example 1-1 and Example 1-2>

Compositions for UV absorption were manufactured under the same conditions as in Example 1 except that in Example 1, the components were prepared so as to have the concentrations given in Table 1.

### <Example 2>

A composition for UV absorption was manufactured under the same conditions as in Example 1 except that in Example 1, an aqueous dispersion of carbon quantum dots (FUJI QD CARBON 308 manufactured by Fuji Pigment Co., Ltd., QD concentration: 15 w/w%) was used instead of the aqueous dispersion of graphene quantum dots (FUJI QD GRAPHENE 818 manufactured by Fuji Pigment Co., Ltd., QD concentration: 15 w/w%).

### <Example 3>

A composition for UV absorption was manufactured under the same conditions as in Example 1 except that in Example 1, a surfactant (KAO AKYPO RLM-100NV (anionic surfactant) manufactured by Kao Corporation) was used instead of the surfactant (RHEODOL AO-10V (nonionic surfactant) manufactured by Kao Corporation).

### <Example 4>

A composition for UV absorption was manufactured under the same conditions as in Example 1 except that in Example 1, a surfactant (ACETAMIN 86 (cationic surfactant) manufactured by Kao Corporation) was used instead of the surfactant (RHEODOL AO-10V (nonionic surfactant) manufactured by Kao Corporation).

### <Example 5>

A composition for UV absorption was manufactured under the same conditions as in Example 1 except that in Example 1, the surfactant (RHEODOL AO-10V (nonionic surfactant) manufactured by Kao Corporation) was not used and glycerin (commercially available as Glycerin from FUJIFILM Wako Pure Chemical Corporation) was used instead of the petrolatum (LOSHI BABY WASERIN Pure N manufactured by Cosmetex Roland Co., Ltd.).

### <Example 5-1 and Example 5-2>

Compositions for UV absorption were manufactured under the same conditions as in Example 5 except that in Example 5, the components were prepared so as to have the concentrations given in Table 2.

### <Example 6>

A composition for UV absorption was manufactured under the same conditions as in Example 5 except that in Example 5, ethoxy diglycol (commercially available as 2-(2-Ethoxyethoxy)ethanol from FUJIFILM Wako Pure Chemical Corporation) was used instead of the glycerin (commercially available as Glycerin from FUJIFILM Wako Pure Chemical Corporation).

### <Example 7>

A composition for UV absorption was manufactured under the same conditions as in Example 5 except that in Example 5, ethanol (commercially available as Ethanol from FUJIFILM Wako Pure Chemical Corporation) was used instead of the glycerin (commercially available as Glycerin from FUJIFILM Wako Corporation).

### <Example 8>

A composition for UV absorption was manufactured under the same conditions as in Example 1 except that in Example 1, the amounts of graphene quantum dots, water, and the surfactant (RHEODOL AO-10V (nonionic surfactant) manufactured by Kao Corporation) were increased and the amount of the petrolatum was reduced so as to be the amounts given in Table 1.

### <Comparative Example 1>

A composition for UV absorption was manufactured under the same conditions as in Example 1 except that in Example 1, the surfactant (RHEODOL AO-10V (nonionic surfactant) manufactured by Kao Corporation) was not used.

### <Comparative Example 2>

A composition for UV absorption was manufactured under the same conditions as in Example 1 except that in Example 1, water containing no carbon-based quantum dots was used.

**[Table 1]**

| | Example 1 | Example 1-1 | Example 1-2 | Example 2 | Example 3 | Example 4 | Example 8 | Comparative Example 1 | Comparative Example 2 |
|---|---|---|---|---|---|---|---|---|---|
| Type of QD | Graphene | Graphene | Graphene | Carbon | Graphene | Graphene | Graphene | Graphene | - |
| w/w% of QD (based on overall amount of composition) | 0.15 | 0.3 | 3 | 0.15 | 0.15 | 0.15 | 6 | 0.15 | - |
| Water | ○ | ○ | ○ | ○ | ○ | ○ | ○ | ○ | ○ |
| w/w% of water (based on overall amount of composition) | 10 | 10 | 20 | 10 | 10 | 10 | 40 | 10 | 10 |
| Surfactant | "RHEODOL AO-10V" manufactured by Kao Corporation | "RHEODOL AO-10V" manufactured by Kao Corporation | "RHEODOL AO-10V" manufactured by Kao Corporation | "RHEODOL AO-10V" manufactured by Kao Corporation | "KAO AKYPO RLM-100NV" manufactured by Kao Corporation | "ACETAMIN 86" manufactured by Kao Corporation | "RHEODOL AO-10V" manufactured by Kao Corporation | (none) | "RHEODOL AO-10V" manufactured by Kao Corporation |
| Type of surfactant | Nonionic | Nonionic | Nonionic | Nonionic | Anionic | Cationic | Nonionic | - | Nonionic |
| w/w% of surfactant (based on overall amount of composition) | 1 | 1 | 2 | 1 | 1 | 1 | 4 | - | 1 |
| Oily base | Petrolatum | Petrolatum | Petrolatum | Petrolatum | Petrolatum | Petrolatum | Petrolatum | Petrolatum | Petrolatum |
| w/w% of oily base (based on overall amount of composition) | 88.85 | 88.7 | 75 | 88.85 | 88.85 | 88.85 | 53 | 88.85 | 89 |
| Dispersibility (immediately after filling) | ○ | ○ | ○ | ○ | ○ | ○ | ○ | × | ○ |
| Dispersibility (1 month after filling) | ○ | ○ | ○ | ○ | ○ | ○ | ○ | × | ○ |
| UV absorption function | ○ | ○ | ○ | ○ | ○ | ○ | ○ | - | × |

**[Table 2]**

| | Example 5 | Example 5-1 | Example 5-2 | Example 6 | Example 7 |
|---|---|---|---|---|---|
| Type of QD | Graphene | Graphene | Graphene | Graphene | Graphene |
| w/w% of QD (based on overall amount of composition) | 0.15 | 0.3 | 3 | 0.15 | 0.15 |
| Water | ○ | ○ | ○ | ○ | ○ |
| w/w% of water (based on overall amount of composition) | 10 | 10 | 10 | 10 | 10 |
| Hydrophilic base | Glycerin | Glycerin | Glycerin | Ethoxy diglycol | Ethanol |
| w/w% of hydrophilic base (based on overall amount of composition) | 89.85 | 89.7 | 87 | 89.85 | 89.85 |
| Dispersibility (immediately after filling) | ○ | ○ | ○ | ○ | ○ |
| Dispersibility (1 month after filling) | ○ | ○ | ○ | ○ | ○ |
| UV absorption function | ○ | ○ | ○ | ○ | ○ |

The evaluation results are shown in Tables 1 and 2.

In Example 1, a composition superior in both dispersibility and UV absorption function was obtained as shown in Table 1. Specifically, through the visual confirmation of the dispersibility of Example 1, it was found as shown in Figs. 1 and 2 that the dispersibility was good both immediately after the filling (Fig. 1) and one month after the filling (Fig. 2), and the whole of the sample was in a uniform state and remained in that state. This is considered to be caused by the surfactant action derived from the addition of the surfactant though petrolatum is oily and the aqueous QD dispersion is hydrophilic, and therefore the petrolatum and the QD aqueous dispersion are immiscible with each other as they are. In addition, it has been found that when the dispersion state is good, a superior UV absorption function, particularly a function of absorbing UVA (wavelength: 315 to 380 nm) and UVB (wavelength: 280 to 315 nm) can be exhibited. On the other hand, in Comparative Example 1, a surfactant was not added, and as a result, the visual confirmation of the dispersibility has revealed that precipitation already occurred immediately after the filling, the dispersibility was poor, and the whole of the sample did not go into a uniform state as shown in Fig. 3. Also one month after the filling, precipitation occurred in the same manner as immediately after the filling (not photographed). In addition, the absorbance of Comparative Example 1 in Table 1 is "-" (bar), but as described above, complete precipitate occurred, so that it is considered that the sample clearly does not have a UV absorption function even if measured. Therefore, comparison between Example 1 and Comparative Example 1 showed that addition of a surfactant is necessary in order to have superior dispersibility and a superior UV absorption function when an oily base is used.

Next, as shown in Table 1, the type of quantum dots was changed from graphene to carbon in Example 2, but a composition having the same performance as in Example 1 was obtained. In each of Example 3 and Example 4, the surfactant was changed from a nonionic surfactant to an anionic surfactant and a cationic surfactant, respectively, but it has been found that even when the type of surfactant was changed, compositions having a UV absorption function while maintaining good dispersibility were obtained. In Example 8, it has been shown that thanks to the addition of a surfactant in an amount of about 1/10 of the added amount of water, a composition having a UV absorption function while maintaining dispersibility was obtained even when the amount of the carbon-based quantum dots was increased. On the other hand, in Comparative Example 2, no carbon-based quantum dots were added. Comparison between Example 1 and Comparative Example 2 has shown that the presence of carbon-based quantum dots was necessary to have a superior UV absorption function. The above facts have shown that to obtain a composition superior in both UV absorption function and dispersibility using an oily base, the presence of a carbon-based quantum dot dispersion (solvent: aqueous solvent) and a surfactant is necessary. Although petrolatum was used for all the oily bases in Examples and Comparative Examples described in Table 1, since the aqueous dispersion of QD can be dispersed in a base with an extremely low polarity such as petrolatum, it is considered that any base having a higher polarity allows easy dispersed in the base.

On the other hand, in Fig. 4, the aqueous QD dispersion used in Example 1 (QD concentration: 1.5 w/w%) was further diluted with purified water into the QD concentrations of 0.0025 w/w% to 0.10 w/w% indicated in the graph, and change in absorption at various QD concentrations was examined. It has been confirmed that a desired UV absorption effect can be exhibited at what QD concentration at the time of manufacturing a preparation. As a result, the change in absorption at a wavelength of 300 nm to 400 nm has shown that the QD concentration of the aqueous QD dispersion is preferably 0.01 w/w% or more. In addition, although organic UV absorbers used in commercially available sunscreens and the like cannot cover a wide range of wavelengths by a single component, it has been found that use of the QD in the present invention allows a wide range of wavelengths such as 300 nm to 400 nm to be covered without blending a plurality of components.

Fig. 5 is a graph showing the result measured as follows. The aqueous QD dispersion (QD concentration: 1.5 w/w%) used in Example 1 was processed to adjust the amount of petrolatum such that the final QD concentration reached the concentrations of 0.15 w/w% to 3 w/w%, which are given in the graph (in the case of 3 w/w%, a QD concentration of 15 w/w% was used without temporarily achieving a QD concentration of 1.5 w/w%), whereby samples were prepared. Each of the samples was applied to a polyethylene film so as to have a thickness of about 25 µm, followed by drying, and then measured with the measuring device and conditions disclosed above (corresponding to Examples 1, 1-1, and 1-2). As a control, petrolatum alone was measured in the same manner. As a result, it has been found that the absorbance was higher in the case of containing graphene quantum dots than in the case of petrolatum alone. In Fig. 5, it has been found that at all of the QD concentrations, a UV absorption function of 3.0 or more was obtained at a wavelength of 350 nm, which is in the UVA range, when the absorbance of petrolatum (control) was 1, and a UV absorption function of 3.0 or more was obtained also at a wavelength of 300 nm, which is in the UVB range, when the absorbance of petrolatum (control) was 1.

Fig. 6 is similar to Fig. 5, and the absorption spectrum was measured for a sample having lapsed 2 months (corresponding to Examples 1, 1-1, and 1-2). The absorption spectrum of the sample having lapsed 2 months is a measurement result of a sample stored without sealing or the like although the sample is in a state in which foreign matters such as dust do not adhere in the state of the preparation thereof. Absorption of 0.5 to 1.0 was confirmed in the range of 300 nm to 450 nm, and deterioration of the UV absorption action was not observed. In particular, at 400 nm or more, the absorption was larger than that at the time of the preparation, but it is considered that this is because moisture was removed and the QD concentration was increased because sealing had not been performed. Although the measured value is not described, it is confirmed that the weight of the sample after a lapse of 2 months has decreased as compared with the sample at the time of preparation.

Next, as shown in Table 2, investigations were conducted using a hydrophilic base. In Example 5, a composition superior in both dispersibility and UV absorption function was obtained. In Examples 6 and 7, the hydrophilic base was changed from glycerin to ethoxy diglycol and ethanol, respectively, but compositions maintaining dispersibility and UV absorption function were obtained. The above facts have suggested that even if an oily base is exchanged to a hydrophilic base, the UV absorption function of QD is not destroyed, and even when various types (Examples 5, 6 and 7) or various concentrations (Examples 5, 5-1 and 5-2) of the hydrophilic base are examined, neither dispersibility nor UV absorption function is affected.

## Claims

1. A composition for UV absorption comprising a carbon-based quantum dot, an aqueous solvent, a surfactant, and an oily base.

2. A composition for UV absorption comprising a carbon-based quantum dot, an aqueous solvent, and a hydrophilic base.

3. The composition for UV absorption according to claim 1 or 2, wherein a content of the carbon-based quantum dot is 0.001 to 10 w/w% based on an overall amount of the composition.

4. The composition for UV absorption according to claim 1, wherein the surfactant is one or more members selected from the group consisting of nonionic surfactant, anionic surfactant, cationic surfactant, and amphoteric surfactant.

5. The composition for UV absorption according to claim 1, wherein the oily base is one or more members selected from the group consisting of hydrocarbons, fatty acids, higher alcohol, ester oil, fat and oil, wax, siloxane, and silicone.

6. The composition for UV absorption according to claim 2, wherein the hydrophilic base is one or more members selected from the group consisting of saccharides, water-soluble polymer, polyhydric alcohol, cellulose derivative, glycol ether, and lower alcohol.

7. A composition for UV absorption, wherein a form of a preparation into which the composition for UV absorption according to claim 1 or 2 is processed is one or more selected from the group consisting of solution, suspension, emulsion, cream, ointment, gel, liniment, spray, aerosol, cataplasm, sheet, powder, and lotion.

8. A cosmetic comprising the composition for UV absorption according to claim 1 or 2.

9. A method for manufacturing a composition for UV absorption, comprising a step of mixing an aqueous dispersion containing a carbon-based quantum dot and an aqueous solvent, a surfactant, and an oily base.

10. A method for manufacturing a composition for UV absorption, comprising a step of mixing an aqueous dispersion containing a carbon-based quantum dot and an aqueous solvent, and a hydrophilic base.

11. A UV absorption method using a composition for UV absorption containing a carbon-based quantum dot, an aqueous solvent, a surfactant, and an oily base.

12. A UV absorption method using a composition for UV absorption containing a carbon-based quantum dot, an aqueous solvent, and a hydrophilic base.
